# EUROPEAN PATENT APPLICATION

(11) **EP 3 937 180 A1**
(43) Date of publication of application: **12.01.2022**
(21) Application number: 20184647.4
(22) Date of filing: 08.07.2020
(51) Int. Cl.: G16H 10/20, G16H 40/63, G16H 40/67, G16H 70/20

(54) **CHANGING BEHAVIOR AFFECTING SLEEP**

(71) Applicant: Koninklijke Philips N.V., 5656 AG Eindhoven (NL)
(72) Inventor: VELTHOVEN, Nathalie Magali Danielle Dessaud, 5656 AE Eindhoven (NL); SARTOR, Francesco, 5656 AE Eindhoven (NL); WEYSEN, Tim, 5656 AE Eindhoven (NL)
(74) Representative: Philips Intellectual Property & Standards

(57) **Abstract**

According to an aspect, there is provided a computer-implemented method of determining a change to be made to a behavior affecting sleep of a user, the method comprising receiving, from at least a first sensor (102) or via a user input, first behavior data (104) relating to a behavior of the user that is to be changed, wherein the behavior affects the user's sleep; receiving, from at least a second sensor (106) or via a user input, first sleep data (108) indicative of a quality of sleep experienced by the user; receiving user data (110) indicative of at least one physiological parameter relating to the user; and determining, based on the first behavior data (104), the first sleep data (108) and the user data (110), a behavior change program (120) including a change to be made to the behavior of the user and a duration and frequency of each of a plurality of intervals during which the behavior change is to be implemented by the user.

## Description

### FIELD OF THE INVENTION

The invention relates to changing a behavior relating to sleep and, more particularly, to determining a change to be made to behavior affecting a person's sleep.

### BACKGROUND OF THE INVENTION

Several factors are known to affect a person's sleep. For example, substances such as caffeine and alcohol and/or behaviors such as using a computer screen, eating a large meal or taking part in physical activity shortly before attempting to fall asleep may affect a person's ability to get to sleep and may affect the quality of sleep experienced. The effect of each factor on the person's sleep depends partly on the homeostasis state of the person, which relates to the level of a particular factor that the person is used to, and the effect that will result from changing that level. For example, if a person regularly consumes 10 cups of caffeinated coffee each day, then the influence of this much caffeine on the person's sleep is likely to be less than if the person regularly consumes just two cups of caffeinated coffee each day, then starts to consume 10 cups. Depending on a person's homeostasis state, changing the caffeine intake may have an impact not only on a person's sleep, but also on the way that person functions while they are awake, sometimes referred to as "daytime functioning".

Bringing about a change in a person's behavior with regard to activities that an effect their sleep can be difficult. One way of motivating change is to enable a person to experience how a particular behavior (or a change in a behavior) impacts the person positively or negatively, and this can help to encourage or discourage a behavioral change. Such a behavioral change technique is sometimes referred to as "awareness creation by experimenting", or "conscious awareness", and can been used to help people to change their behavior in order to improve their health. However, some people may have a homeostasis state that corresponds to excessive consumption or intake, such that experiencing a change to that homeostasis state may have little impact or may have a negative impact. For example, if a person is used to drinking 10 cups of coffee each day, then reducing the intake may have a negative impact, an impact that is barely noticeable, or an impact that is not immediately evident. Any of these can be demotivating for a person, making the behavioral change even harder to effect.

In cases where a person has a particular behavior that is very regular or even habitual, then making changes to that behavior may have a low impact on that person's sleep, and in some cases may even have a negative effect, for example due to the withdrawal effect. It is also particularly difficult to change a person's behavior if that behavior has become a way of life for the person.

There is, therefore, a desire for a technique that can be used to effectively bring about behavioral change in a person improve their sleep.

### SUMMARY OF THE INVENTION

It can be difficult to bring about change of a person's behavior, particularly when the behavior to be changed forms a significant part of the person's lifestyle. Various behavioral change techniques are used across various fields and, depending on the behavior to be changed, some techniques are more effective than others. One behavioral change technique that has been recognized by the inventors as being particularly beneficial is interval training. Interval training is used in health and fitness training regimes to bring about significant changes in a person's fitness levels with relatively low input or exertion. In essence, a person performs physical activity (e.g. exercise) in intervals; typically high levels of intense exercise for a short period of time, followed by a short rest to allow for recovery. The short intervals of exercise and rest are repeated according to a training plan. Training in this way is known to be very effective.

The inventors of the present disclosure have recognized that interval training techniques can also be effective in bringing about changes in behavior affecting a person's sleep. For example, for a person whose sleep is affected by the regular consumption of 10 cups of coffee, and interval-based change in behavior (reducing the coffee intake gradually in intervals) may be more effective (and acceptable to the person who is in behaviors to change) than a major change, such as attempting to cut out coffee altogether.

Embodiments of the present invention seek to address at least some of the above-identified problems with behavioral change techniques by determining a behavior change program for a person, which includes a series of intervals over which the behavior change is to be introduced. The behavior change program is determined based on information relating to the person (referred to herein as a user), and on other information such as information relating to the behavior that is to be changed (e.g. coffee consumption) and information relating to the amount and/or quality of the user's sleep.

According to a first specific aspect, there is provided a computer-implemented method of determining a change to be made to a behavior affecting sleep of a user, the method comprising receiving, from at least a first sensor or via a user input, first behavior data relating to a behavior of the user that is to be changed, wherein the behavior affects the user's sleep; receiving, from at least a second sensor or via a user input, first sleep data indicative of a quality of sleep experienced by the user; receiving user data indicative of at least one physiological parameter relating to the user; and determining, based on the first behavior data, the first sleep data and the user data, a behavior change program including a change to be made to the behavior of the user and a duration and frequency of each of a plurality of intervals during which the behavior change is to be implemented by the user.

In some embodiments, the method may further comprise providing the behavior change program for display to the user; receiving, from at least the first sensor or via a user input, second behavior data relating to a behavior of the user following the provision of the behavior change program; determining, based on the second behavior data, a level of adherence to the behavior change program by the user; and determining, based on the level of adherence, a modification to be made to the behavior change program to increase the level of adherence of the user.

The method may, in some embodiments, further comprise receiving daytime functioning data indicative of a quality of daytime functioning by the user; receiving, via a user input or from the second sensor, second sleep data indicative of a quality of sleep experienced by the user following the provision of the behavior change program; and determining, based on the daytime functioning data and at least one of the first sleep data and the second sleep data, a user awareness measure indicative of an awareness of the user of an impact of the behavior change program.

In some embodiments, the method may comprise determining, based on the level of adherence and the user awareness measure, an indication of the user's susceptibility to behavioral change; and determining, based on the second sleep data and the second behavior data, a user sensitivity level, indicating a sensitivity of the user to the behavior affecting sleep.

Determining a modification to be made to the behavior change program may be further based on the indication of the user's susceptibility to behavioral change and the user sensitivity level. In some embodiments, determining a modification to be made to the behavior change program may be repeated after a defined number of days.

The method may, in some embodiments, further comprise, responsive to determining that level of adherence is below a defined threshold, generating a request to be displayed to the user prompting the user to perform a defined activity.

In some embodiments, the defined activity may be configured to measure daytime functioning data indicative of a quality of daytime functioning by the user.

The method may, in some embodiments, further comprise obtaining data indicative of domain knowledge relating to behavior change techniques. Determining a behavior change program may further be based on the domain knowledge data.

According to a second aspect, there is provided a computer program product comprising a non-transitory computer-readable medium, the computer-readable medium having computer-readable code embodied therein, the computer-readable code being configured such that, on execution by a suitable computer or processor, the computer or processor is caused to perform steps of the methods disclosed herein.

According to a third aspect, there is provided an apparatus for determining a change to be made to a behavior affecting sleep of a user. The apparatus comprises a processor configured to receive, from at least a first sensor or via a user input, first behavior data relating to a behavior of the user that is to be changed, wherein the behavior affects the user's sleep; receive, from at least a second sensor or via a user input, sleep data indicative of a quality of sleep experienced by the user; receive user data indicative of at least one physiological parameter relating to the user; and determine, based on the first behavior data, the sleep data and the user data, a behavior change program including a change to be made to the behavior of the user and a duration and frequency of each of a plurality of intervals during which the behavior change is to be implemented by the user.

In some embodiments, the processor may be further configured to provide the behavior change program for display to the user; receive, from at least the first sensor or via a user input, second behavior data relating to a behavior of the user following the provision of the behavior change program;
determine, based on the second behavior data, a level of adherence to the behavior change program by the user; and determine, based on the level of adherence, a modification to be made to the behavior change program to increase the level of adherence of the user.

The processor may, in some embodiments, be further configured to receive daytime functioning data indicative of a quality of daytime functioning by the user; receive, via a user input, second sleep data indicative of a quality of sleep experienced by the user following the provision of the behavior change program; and determine, based on the daytime functioning data and at least one of the first sleep and the second sleep data, a user awareness measure indicative of an awareness of the user of an impact of the behavior change program.

In some embodiments, the processor may be further configured to determine, based on the level or adherence and the user awareness measure, an indication of the user's susceptibility to behavioral change; and determine, based on sleep data from the second sensor or via the user input and the daytime functioning data, a user sensitivity level, indicating a sensitivity of the user to the behavior affecting sleep.

According to a fourth aspect, there is provided a system for determining a change to be made in a behavior affecting sleep of the user. The system comprises a first sensor configured to measure first behavior data relating to a behavior of the user that is to be changed, wherein the behavior affects the user's sleep; a second sensor configured to measure sleep data indicative of a quality of sleep experienced by the user; a display apparatus; and an apparatus as disclosed herein.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a flow diagram representing a system for determining a behavioral change to be made;
Fig. 2 is a flowchart of an example of a method of determining a behavioral change to be made;
Fig. 3 is a flowchart of a further example of a method of determining a behavioral change to be made;
Fig. 4 is a schematic illustration of an example of a processor in communication with a computer-readable medium;
Fig. 5 is a schematic illustration of an example of an apparatus for determining a behavioral change to be made;
Fig. 6 is a schematic illustration of an example of a system for determining a behavioral change to be made;
Fig. 7 is a graph showing how impact varies as a function of time for three different weeks; and
Fig. 8 is a graph showing how the impact varies as a function of time over various intervals.

### DETAILED DESCRIPTION OF EMBODIMENTS

Embodiments of the present disclosure make use of various pieces of data relating to a person or user in order to develop a behavior change program intended to motivate, encourage and bring about change in the behavior of a user to improve the amount and/or quality of sleep experienced by the user. Specifically, the behavior or behaviors is to be changed may be behaviors that affect sleep. In this disclosure, one example of a sleep-affecting behavior that is discussed is the consumption of caffeinated drinks, such as coffee. Consuming large amounts of coffee, particularly in the evening, or shortly before attempting to sleep, may adversely affect a person's ability to get to sleep, and/or may adversely affect the quality of sleep experienced by the person. The present disclosure is also relevant to other sleep-affecting behaviors. For example, alcohol consumption may affect the quality of sleep experienced by a person, while the alcohol remains in the person's system; consuming a large amount of food (e.g. eating a large meal) close to bedtime may make it more difficult for a person to fall asleep; taking exercise (particularly vigorous exercise) close to bedtime may stimulate a person's body and brain, thereby making it more difficult for that person for sleep; ambient conditions such as noise, light and temperature may also affect a person's ability to fall asleep, and the quality of the sleep; medication such as sleep-enhancing medication or medication that contains caffeine may affect the person's sleep and/or the functioning of the person while they are awake; the use of computer equipment such as smart phones and tablet computers and watching television (sometimes referred to as screen time) may also affect a person's ability to fall asleep and/or to sleep soundly. Embodiments of the present disclosure may be used to encourage or bring about change of these and/or other behaviors that are known to affect sleep.

The behavioral change techniques disclosed herein are intended to encourage and motivate a person to adhere to a changed behavior so that the person is not discouraged from maintaining a behavioral change because any negative impact of the change outweighs the positive impact on the person's sleep. The techniques disclosed herein can be used to generate a personalized program or plan based on an interval training technique that can modify the homeostatic state of the person towards a desired or intended behavior (e.g. reducing caffeine intake). In some examples, this may be achieved by increasing the perceived sensitivity of the person to a behavior, thereby enhancing their susceptibility to change the behavior. Using the disclosed technique, behavior change can be brought about with relatively little effort for the user, and a relatively fast impact (e.g. a quick change in the behavior).

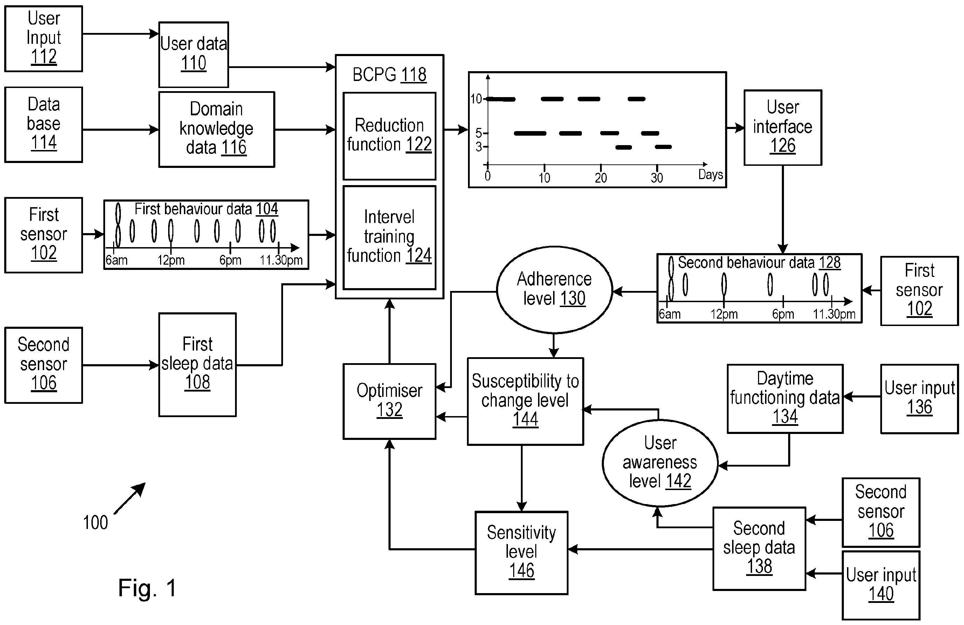

Referring to the drawings, Fig. 1 is a flow diagram showing the movement of data through a system 100 that may be used to determine a change to be made to a behavior affecting sleep of a user. As will become apparent from this disclosure, at least some of the components shown in Fig. 1 may be implemented using a processor or processors.

The system 100 of Fig. 1 includes a first sensor 102 configured to measure or capture first behavior data 104 relating to a user's behavior that is to be changed. The behavior to be changed may comprise any of the behaviors are discussed above and, specifically, may comprise a behavior affecting sleep of the user, including the ability of the user to fall asleep and the quality of sleep experienced by the user. In an example where the behavior to be changed relates to caffeine intake in cups of coffee, the first sensor 102 may, for example, comprise a sensor built into a coffee machine, capable of measuring an amount of coffee, or a number of cups of coffee, distributed by the coffee machine to the user. In other examples, the first sensor 102 may comprise a user-operated sensor (e.g. a tapping device) via which the user can keep count of the number of cups of coffee consumed each day. In some cases, it may not be possible to capture or measure the first behavior data 104 using just the sensor 102 and, in such examples, the first behavior data may be generated or supplemented by a user providing details of the amount of per day. For example, the user may keep a diary to keep note of the coffee consumption levels. The first behavior data 104 may, for example, include a number of cups of caffeinated coffee consumed within each hour of a particular day.

The system 100 also includes a second sensor 106 configured to measure or capture sleep data 108 indicative of a quality of sleep experienced by the user. The sleep data 108 may, for example, include data indicative of a time that the user fell asleep, an amount of time that it took the user to fall asleep, the amount of time that the user spent asleep, and details of any interruptions to the sleep experienced by the user. The second sensor 106 may, for example, comprise a sensor built into or forming part of a wearable device, such as a smartwatch, bracelet, chest strap, or the like, a pulse oximeter (e.g. configured to generate a photoplethysmogram (PPG)) and/or a sensor associated with the user's bed or mattress, configured to measure movement and/or breathing patterns while the user sleeps. In some cases, the sleep data 108 may comprise, or be supplemented by data provided by the user, such as data in which the user indicates a quality of sleep (e.g. on a numerical scale of 1 to 10). For example, the user may keep a sleep diary to track their sleep each night.

The system 100 may also make use of user data 110, indicative of at least one physiological parameter relating to the user. For example, parameters of the user, such as their gender, age, height, weight, and the like, may be included in the user data 110 for use in the system 100. The user data 110 may be provided in the form of a user input 112, for example via a user interface, or retrieved from a storage (e.g. a memory, not shown) storing data relating to the user or to multiple users, for example in the form of medical records.

A database 114 may be accessible by or form part of the system 100, and may be used to store various data such as domain knowledge and data 116. The domain knowledge data 116 may include data and information familiar to those skilled in the field of behavioral change techniques (e.g. experts in the field) and may include logic to be used by the system 100 when determining how to change a user's behavior. For example, the domain knowledge data 116 may include logic indicating that reducing caffeine intake close to bedtime as the greatest effect on improving sleep, and/or may include logic proposing a reduction of caffeine intake of at least 50% during a first interval (i.e. a first period during which the behavioral change is made) in cases where the user consumes eight or more cups of coffee per day. In some examples, the domain knowledge data 116 may include, or may be acquired or extracted from scientific literature.

According to various examples, the first behavior data 104, the sleep data 108 and the user data 110 may be provided to a behavior change program generator (BCPG) 118. The BCPG 118 may be implemented by a processor. For example, the BCPG 118 may comprise a computer program or application that is implemented by the processor executing code. The BCPG 118 may use the first behavior data 104, the sleep data 108 and the user data 110 to determine or generate a behavior change program 120 for the user. The behavior change program 120 includes a change to be made to the behavior of the user and a duration and frequency of each of a plurality of intervals during which the behavior change is to be implemented by the user. In some examples, the BCPG 118 may determine the change to be made to the user's behavior using a first module or function referred to as a reduction function 122, and may determine the duration and frequency of each of the plurality of intervals using a second module or function referred to as an interval training function 124. In some examples, the reduction function 122 may additionally use the domain knowledge data 116 to determine the change to be made to the user's behavior.

The reduction function 122 provides as its output a proposed behavioral change that it is intended should be made by the user. The change to the behavior may include an indication of what should change (e.g. the number of cups of coffee consumed each day), the amount by which it should change (e.g. reduce the coffee intake by 50%) and when the change should be made (e.g. reduce the coffee intake after 6pm by 100%, and reduce the coffee intake between 1pm and 6pm by 60%).

The interval training function 124 provides as its output an indication of how the proposed behavioral change should be varied over time to achieve an optimal impact. In other words, the interval training function 124 determines a series of intervals during which the behavioral change is to be implemented to some extent. The interval training function 124 may determine a duration (e.g. one day) and frequency (e.g. every two days, or every other day) of each of a plurality of intervals during which the behavior change is to be implemented by the user. In some examples, the interval training function 124 may determine that the behavior change is to be implemented for an interval of a particular duration (e.g. one day of consuming 50% of the user's regular amount of coffee) then no change to the behavior is to be implemented for a particular duration (e.g. one day of consuming the user's regular amount coffee). The interval training function 124 may also determine that this pattern (e.g. one day of changed behavior followed by one day of unchanged behavior) is to be repeated for a defined period (e.g. one week). In another example, where the behavior relates to alcohol intake, the interval training function 124 may determine that, for 14 days, the change to the user's behavior should include alternating between one day of the user's normal alcohol intake and 2 days of a reduced alcohol intake, with a different arrangement from day 15. For example, the reduction function 122 may determine that the alcohol intake should be reduced by 80%, and the interval training function 124 may determine that the user should aim to consume alcohol at the reduced levels for two out of three days for 14 days. The exact change (e.g. the reduction in coffee consumption or the reduction in alcohol intake) and the exact pattern of intervals over which the change should be implemented are determined by the reduction function 122 and the interval training function 124 based on the data provided to the BCPG 118 and may, therefore, differ from user to user.

The behavior change program 120 represents advised behavior for the user that should be followed in order for the user to improve their sleep. The behavior change program 120 may be presented to the user via a user interface or display 126. The display 126 may form part of a computer device, such as a smartwatch, a smart phone, a tablet computer, laptop computer, a desktop computer, an interactive mirror, and the like. For example, the behavior change program 120 may be presented on the display 126 in the form of a textual message (e.g. "try not to drink any more coffee today") or in the form of an image or graph, as illustrated by the behavior change program 120 of Fig. 1.

Once the behavior change program 120 has been displayed to the user, it is intended that the user follows the behavior change program so as to change the sleep-affecting behavior and improve their sleep. In some embodiments, the system 100 may include a feedback mechanism to enable the effect or impact of the behavior change program 120 on the user, such that, if necessary, changes to the behavior change program can be made.

After the behavior change program 120 has been presented to the user, the first sensor 102 may be used to measure or acquire second behavior data 128 relating to the behavior of the user following the provision of the behavior change program 120. In other words, if the user is following the behavior change program 120, then the second behavior data 128 is representative of the changed behavior the user which, in the example given above, should represent a reduced level of coffee consumption. As with the first behavior data 104, the second behavior data 128 may comprise a frequency of a particular behavior (e.g. a number of cups of coffee consumed over a particular period of time). In addition or alternatively to data from the first sensor 102 (e.g. a counter of a coffee machine), the second behavior data 128 may include data provided via a user input, such as a record of diary keeping track of their behavior (e.g. a diary keeping note of the number of coffees consumed each day).

Based on the second behavior data 128, it is possible to determine a level of adherence 130 which represents the adherence of the user to the behavior change program 120. The level of adherence 130 may, for example, be represented in the form of a percentage, indicating how well the user has kept to the advised behavior set out in the behavior change program 120. For example, if the user has changed their behavior in accordance with the behavior change program 120 (e.g. by reducing the number of coffees consumed on the indicated days), then the level of adherence 130 will be 100%. The adherence level 130 may be determined using an adherence analyzer which compares the advised behavior set out in the behavior change program 120 with the user's actual behavior, set out in the second behavior data 128.

In some embodiments, the level of adherence 130 may be provided to an optimizer 132, which may provide an input into, or work in conjunction with, the BCPG 118 to modify the behavior change program 120. The optimizer 132 may, for example, modify the reduction function 122 and/or the interval training function 124 so that the advised behavior generated as part of the behavior change program 120 is different. The role of the optimizer 132 may be to make modifications such that the user is more likely to adhere to the behavior change program 120. In other words, the optimizer 132 may aim to modify the behavior change program 120 so as to increase the level of adherence 130. While, in some embodiments, the optimizer 132 may make necessary changes based on the level of adherence 130 and the second behavior data 128, in other embodiments, additional data may be used so that the optimizer can change the behavior change program 120 to further improve the level of adherence 130. In some embodiments, the optimizer 132 may be implemented using one or more rules or algorithms while, in other embodiments, the optimizer may make use of machine learning techniques (e.g. a trained machine learning model) to optimise the behavior change program 120 effectively.

In some embodiments, daytime functioning data 134 may be measured or acquired in respect of the user. Daytime functioning data 134 may represent how the user functions during the day (i.e. during the time that the user is not sleeping), as this may provide an insight as to the effect of the behavior (e.g. coffee consumption) and/or the effect of the changed behavior (e.g. the behavior if the user follows the behavior change program 120) on the user's everyday activities. The daytime functioning data 134 may, for example, include user-provided information relating to their alertness levels, their fatigue levels, and so on. In some embodiments, the daytime functioning data 134 may be provided via a user input 136, in the form of a self-reporting analysis, whereby the user inputs data representing how they are functioning during the day. This may be done in the form of a questionnaire, for example. In other examples, the daytime functioning data 134 may be acquired by asking the user to perform one or more cognitive tests (e.g. via a computing device such as a smart phone). The results of such a cognitive test may be used to determine a level of daytime functioning of the user.

It may also be useful to measure how the behavior change program 120 has affected the user's asleep, and this may be analyzed using the second sleep data 138. The second sleep data 138 may include data indicative of how easily and/or quickly the user falls asleep, how long the user sleeps for and/or a quality of the sleep of the user. The second sleep data 138 may be obtained using the second sensor 106 (e.g. a sensor of a wearable device, a pulse oximeter and/or a sensor associated with the user's bed or mattress) and/or may be provided by the user via a user input 140, for example in the form of a sleep diary.

The daytime functioning data 134 and the second sleep data 138 may together provide an insight into the impact that the change in behavior has had on the user's sleep and on the user's daytime activities. The daytime functioning data 134 and the second sleep data 138 may be used to determine a user awareness measure 142, indicative of the user's awareness of the impact of the behavior change program 120. The user awareness measure 142, which may be referred to as a conscious awareness creation index, is higher when the impact on the subjective sleep experience and the daytime functioning experience of the user is higher. Therefore, it is particularly useful to have daytime functioning data 134 and second sleep data 138 that is provided by the user when determining the user awareness measure 142. However, if such subjective data is not available, then objective data (e.g. from a cognitive test and from the second sensor 106) may be used to determine the user awareness measure 142.

The user awareness measure 142 may be combined with the level of adherence 130 to determine an indication of the user's susceptibility to behavioral change 144, sometimes referred to as a susceptibility to change index. The susceptibility to behavioral change 144 gives an indication of how likely it is that the behavior of the user will be changed in accordance with the behavior change program 120. In some examples, additional data may be used in determining the susceptibility to behavioral change 144 of the user including, for example, answers provided by the user to a questionnaire relating to motivation. Such a questionnaire may relate to the willingness of the user to change their behavior, the ability of the user to change their behavior and/or the self-efficacy of the user. In embodiments where the susceptibility to behavioral change 144 of the user has been determined, the susceptibility to change may be provided to the optimizer 132 and used to modify the behavior change program 120.

The second behavior data 128 and the second sleep data 138 may be used to determine a user sensitivity level 146, indicative of a sensitivity of the user to the behavior affecting their sleep. The user sensitivity level 146, sometimes referred to as a sensitivity index, gives an indication of the sensitivity of the behavior (e.g. coffee consumption) on the user's sleep. This can give a measure of the impact of changing the behavior (e.g. reducing the coffee consumption) on the user's sleep. The user sensitivity level 146 may also be used by the optimizer 132 to modify the behavior change program 120.

The optimizer 132 may use the various inputs available (e.g. the adherence level 130, the susceptibility to change 144 and/or the sensitivity level 146) to modify the reduction function 122 and/or the interval training function 124 of the BCPG 118 to generate the most appropriate behavior change program 120 for the user. In some examples, the optimizer 132 may be used on multiple occasions (e.g. periodically), using regular feedback from the user on the impact of the behavior change program 120 on their behavior, their daytime functioning and their sleep. For example, the optimizer 132 may modify the behavior change program 120 daily, weekly, monthly, or based on some other time period.

The intention of the behavior change program 120 (either as initially determined or following one or more optimizations by the optimizer 132) is that the user changes their behavior affecting their sleep in such a way that the user is not demotivated or discouraged from maintaining the changed behavior. An important aspect relating to the success of changing the user's behavior is in the appropriate determination of intervals by the interval training function 124. The behavior change program 120 aims to increase the user's susceptibility to behavioral change 144 by increasing the user's sensitivity to the particular behavior to be changed and, therefore, increasing the user's awareness of the impact of making that behavioral change (i.e. increasing the user awareness level 142) by implementing the behavior change program 120 the user should be able to move from one homeostatic state to another, thereby changing their regular, everyday behavior in a positive way (e.g. by reducing their coffee consumption levels), leading to an increase in their sleep sensitivity to that behavior.

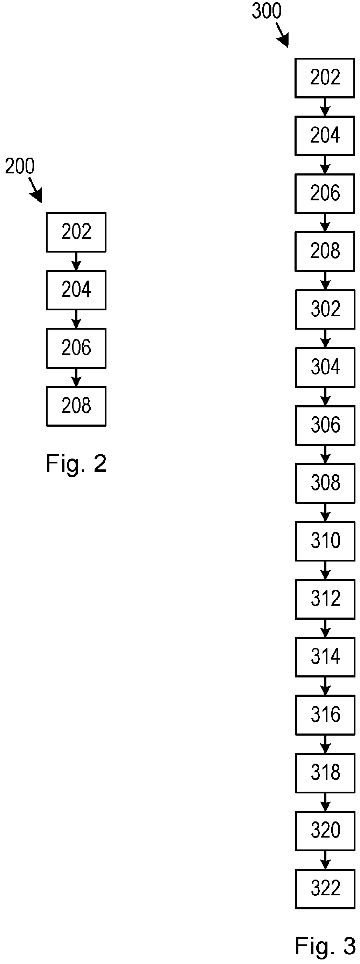

According to a first aspect, the present invention provides a method. Fig. 2 is a flowchart of an example of a method 200 of determining a change to be made to a behavior affecting sleep of a user. In various embodiments, the method 200 may comprise a computer-implemented method. Thus, steps of the method 200 may be performed using one or more processors or processing circuitry, for example forming part of a computing device or distributed computing system.

The method 200 comprises, at step 202, receiving, from at least a first sensor 102 or via a user input, first behavior data 104 relating to a behavior of the user that is to be changed, wherein the behavior affects the user's sleep. As in the example given above, the behavior may, for example, comprise consuming caffeinated coffee. The method 200 comprises, at step 204, receiving, from at least a second sensor 106 or via a user input, first sleep data 108 indicative of a quality of sleep experienced by the user. At step 206, the method 200 comprises receiving user data 110 indicative of at least one physiological parameter relating to the user. The at least one physiological parameter may, for example, include the age, gender, height or weight of the user. The method 200 comprises, at step 208, determining, based on the first behavior data 104, the first sleep data 108 and the user data 110, a behavior change program 120 including a change to be made to the behavior of the user and a duration and frequency of each of a plurality of intervals during which the behavior change is to be implemented by the user. As noted above, the change to be made to the behavior of the user may be determined using a reduction function 122, and the change may be defined in terms of an absolute change (e.g. a reduction of a given number of cups of coffee per day) or as a relative change (e.g. reducing the coffee consumption by 50%) the duration and frequency of each of a plurality of intervals during which the behavior changes to be implemented may be determined using an interval training function 124.

The determination made at step 208 is a user-specific program intended to enable a user to effectively change at least one sleep-affecting behavior. By designing a behavior change program 120 that involves the gradual or staggered introduction of the change through a series of intervals, the user is more likely to adhere to the change in behavior as such a change may yield positive results without the user experiencing a significant negative impact on their everyday life.

Fig. 3 is a flowchart of a further example of a method 300 of determining a change to be made to a behavior affecting sleep of a user. The method 300 may also comprise a computer-implemented method, and may include the steps of the method 200 discussed above. The method 300 may comprise, at step 302, obtaining data indicative of domain knowledge 116 relating to behavior change techniques. The domain knowledge data 116 may, in some examples, be obtained from the database 114, and may include rules and/or logic indicative of known techniques and advice for changing behavior. The domain knowledge data 116 may relate to the general population and/or to certain groups of people, such that relevant data can be obtained, that relates to the user. In examples where domain knowledge data 116 is obtained or available, determining (step 208) the behavior change program 120 may be further based on the domain knowledge data.

At step 304, the method 300 may further comprise providing the behavior change program 120 for display to the user. The behavior change program 120 may for example, be presented to the user using a user interface or display 126 which may form part of a computing device such as a smartphone or tablet computer. At step 306, the method 300 may comprise receiving, from at least the first sensor 102 or via a user input, second behavior data 128 relating to a behavior of the user following the provision of the behavior change program 120. The second behavior data 128 may be indicative of the amount by which the user has changed their behavior in light of the behavior change program 120. The method 300 may comprise, at step 308, determining, based on the second behavior data 128, a level of adherence 130 to the behavior change program 120 by the user. At step 310, the method 300 may comprise determining, based on the level of adherence 130, a modification to be made to the behavior change program 120 to increase the level of adherence of the user. The determination of step 310 may be made using the optimizer 132. For example, the modification to be made may comprise a modification to the reduction function 122 and/or the interval training function 124 such that they output a modified behavior change program 120.

As noted above, additional data may be used to determine the modification (step 310). Thus, the method 300 may comprise, at step 312, receiving daytime functioning data 134 indicative of a quality of daytime functioning by the user. The daytime functioning data 134 may, in some examples, be formatted quantitatively. In some embodiments, the daytime functioning data 134 may be provided via a user input 136 in the form of a subjective view of the user regarding their view of the quality of their daytime functioning following uptake of the behavior change program 120 while, in other embodiments, the daytime functioning data may be obtained in a more objective manner, for example through use of one or more cognitive or physical assessments. At step 314, the method 300 may further comprise receiving, via a user input 140 or from the second sensor 106, second sleep data 138 indicative of a quality of sleep experienced by the user following the provision of the behavior change program 120.

The method 300 may comprise, at step 316, determining, based on the daytime functioning data 134 and at least one of the first sleep data 108 and the second sleep data 108, a user awareness measure 142 indicative of an awareness of the user of an impact of the behavior change program 120. As indicated above, the user awareness measure 142, which may be referred to as the conscious awareness creation index, provides an indication of the impact on the user's sleep experience and on their daytime functioning experience resulting from the implementation of the behavior change program 120.

At step 318, the method 300 may comprise determining based on the level of adherence 310 and the user awareness measure 142, an indication of the user's susceptibility to behavioral change 144. In some embodiments, the method 300 may comprise, at step 320, determining, based on the second sleep data 138 and the second behavior data 128, a user sensitivity level 146, indicating a sensitivity of the user to the behavior affecting sleep. In some embodiments, determining a modification (step 310) to be made to the behavior change program 120 may be further based on the indication of the user's susceptibility to behavioral change 144 and the user sensitivity level 146.

As noted above, the optimizer 132 may use the data provided to it in order to determine modifications to be made to the behavior change program 120, and it may therefore be the optimizer 132 that determines the modification at step 310. The optimizer 132 may function on a periodic basis, modifying the behavior change program 120 at a rate set by an operator or by the user, depending on the behavior to be changed. Thus, determining (step 310) a modification to be made to the behavior change program 120 may be repeated after a defined number of days. For example, in some cases, a modification may be determined daily (or even more frequently than daily), every other day, weekly, biweekly, monthly, quarterly, or the like. In other examples, the modification may not be determined periodically, but rather may be determined on ad hoc basis, for example if it is determined that the user is failing to adhere to the behavior change program 120. Thus, in some embodiments, responsive to determining that the level of adherence 130 is below a defined threshold, the optimizer 132 may be used to determine a modification (step 310) to be made to the behavior change program 120.

In some embodiments, at step 322, responsive to determining that the level of adherence 130 is below a defined threshold, the method 300 may further comprise, generating a request to be displayed to the user prompting the user to perform a defined activity. The defined threshold of the adherence level 130 may be chosen depending on the importance that the user adheres to the behavior change program 120 and, in some cases, the defined threshold may comprise 90%, 75%, 50%, or the like. In other examples, the defined threshold may comprise an absolute value, rather than a percentage. The defined activity that the user is prompted to perform may comprise an activity intended to improve their level of adherence 130. For example, in the case where the user is intending to reduce their consumption of coffee, then the defined activity may comprise an activity such as playing a game or exercising, which might occupy the user and take their mind off the desire to drink coffee. In other embodiments, the defined activity may be configured to measure daytime functioning data 134 indicative of a quality of daytime functioning by the user. For example, the defined activity may comprise a cognitive task or some other assessment to be performed or completed by the user (e.g. using their smart phone), and the output or results of that activity may comprise the daytime functioning data 134 that may be used to determine the user awareness level 142.

Steps of the methods 200, 300 discussed herein may be performed using one or more processors which may form part of all be associated with one or more computing devices. Alternatively, steps of the methods 200, 300 may be performed by processing apparatuses located in one or more servers or computing facilities the form part of a distributed computing network, such as a cloud computing environment.

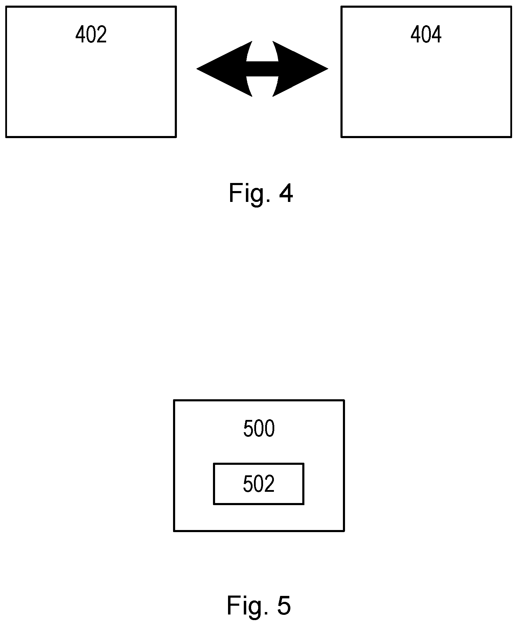

According to a second aspect, the present invention provides a computer program product. Fig. 4 is a schematic illustration of an example of a processor 402 in communication with a computer-readable medium 404. A computer program product may comprise a non-transitory computer-readable medium 404, the computer-readable medium having computer-readable code embodied therein, the computer-readable code being configured such that, on execution by a suitable computer or processor 402, the computer or processor is caused to perform steps of the methods 200, 300 disclosed herein.

According to a third aspect, the present invention provides an apparatus. Fig. 5 is a schematic illustration of an example of an apparatus 500. The apparatus 500 may comprise an apparatus for determining a change to be made to a behavior affecting sleep of a user, and may perform one or more of the steps of the methods 200, 300 disclosed herein. The apparatus 500 comprises a processor 502 configured to receive, from at least a first sensor 102 or via a user input, first behavior data 104 relating to a behavior of the user that is to be changed, wherein the behavior affects the user's sleep. The processor 502 is further configured to receive, from at least a second sensor 106 or via a user input, sleep data 108 indicative of a quality of sleep experienced by the user. The processor 502 is further configured to receive user data 110 indicative of at least one physiological parameter relating to the user. The processor 502 is further configured to determine, based on the first behavior data 104, the sleep data 108 and the user data 110, a behavior change program 120 including a change to be made to the behavior of the user and a duration and frequency of each of a plurality of intervals during which the behavior change is to be implemented by the user.

In some embodiments, the processor 502 of the apparatus 500 may further be configured to acquire data relating to the user following implementation of the behavior change program 120 and feedback into the BCPG 118 so as to modify and/or optimize the behavior change program for the user. Thus, in some embodiments, the processor 502 may be further configured to provide the behavior change program 120 for display to the user (e.g. on the display 126). The processor 502 may be further configured to receive, from at least the first sensor 102 or via a user input, second behavior data 128 relating to a behavior of the user following the provision of the behavior change program 120. The processor 502 may be further configured to determine, based on the second behavior data 128, a level of adherence to the behavior change program 120 by the user. The processor 502 may be further configured to determine, based on the level of adherence 130, a modification to be made to the behavior change program 120 to increase the level of adherence of the user.

The processor 502 may, in some embodiments, be further configured to receive daytime functioning data 134 indicative of a quality of daytime functioning by the user. In some emblems, the processor 502 may be further configured to receive, via a user input 140, second sleep data 138 indicative of a quality of sleep experienced by the user following the provision of the behavior change program 120. The processor 502 may be further configured to determine, based on the daytime functioning data 134 and at least one of the first sleep data 108 and the second sleep data 138, a user awareness measure 142 indicative of an awareness of the user of an impact of the behavior change program 120.

In some embodiments, the processor 502 may be further configured to determine, based on the level of adherence 130 and the user awareness measure 142, an indication of the user's susceptibility to behavioral change 144. The processor 502 may be further configured to determine, based on sleep data 138 from the second sensor 106 and the daytime functioning data 134, a user sensitivity level 146, indicating a sensitivity of the user to the behavior affecting sleep.

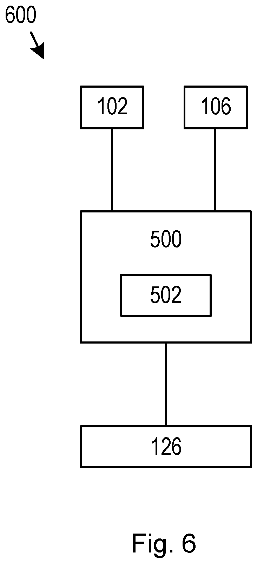

According to a fourth aspect, the present invention provides a system. Fig. 6 is a schematic illustration of an example of a system 600. The system 600 may be considered to be a system for determining a change to be made to a behavior affecting sleep of a user. The system 600 comprises a first sensor 102 configured to measure first behavior data 104 relating to a behavior of the user that is to be changed, wherein the behavior affects the user's sleep. The system 600 also comprises a second sensor 106 configured to measure sleep data 108 indicative of a quality of sleep experienced by the user. The system 600 also comprises a display apparatus 126 and an apparatus 500 as disclosed herein. The components of the system 600 may be connected via a wired or wireless connection and may communicate to one another using any known communication protocol.

### Example

The following is an example showing how the methods, apparatus and system disclosed herein may be implemented. A user may log their caffeine consumption (e.g. via cups of coffee) through the day (or over several days). On average they consume 10 cups of coffee, spread throughout the course of a day as shown for example in the log shown in box 104 of Fig. 1.

Based on the distribution profile of the coffee consumption over the course of the day, the reduction function 122 generates, as part of the behavior change program 120, a recommendation that the user stops drinking any coffee after 6pm and reduces by their total consumption throughout the day by half, by removing caffeine intake events, not reducing doses. In other words, it is recommended that the user continues to consume the same amount/strength of coffee on each occasion, but reduces the number of occasions on which coffee is consumed. Specifically, the reduction function 122 recommendation is to remove one coffee before 12pm, one coffee between 12pm and 6pm, and all three coffees after 6pm. The reduction function 122 is defined based on healthy sleep behavior for a given user profile, and balances between reducing the negative impact of caffeine (i.e. preventing the user from sleeping), but at the same time, limiting any withdrawal effect resulting from removing coffee consumption completely for this user (which could cause a headache and make it difficult for user to maintain the change).

The user is presented with the advised behavior forming part of the behavior change program 120 via the user interface 126 which may, for example, comprise an application with a schedule-like-interface which illustrates to the user blocks indicating where coffee can be consumed and where coffee consumption is not recommended.

Based on this advice, the user monitors their caffeine intake (e.g. quantity and timing) for the next day, and this forms the second behavior data 128. Based on the timing of the removal of coffee intake, the adherence level 130 may be determined. In this example, the adherence level may be estimated using timing-specific adherence points:
Adherence before 12pm= score 1 point
Adherence between 12pm and 6pm: score 2 points
Adherence after 6pm = score 5 points

The adherence points available may be varied from user to user and, specifically, may be varied based on the sensitivity level of the user. The time slots allocated for the adherence points can be defined based on the user's sleep schedule. For example, for a shift worker, 5 adherence points can still be achieved if they cut out a coffee within the six-hour period before the user's regular bedtime.

In this example, if the user reduces their intake by 3 cups to a total of 7 (i.e. 1 cup between 12pm and 6pm and 2 cups after 6pm, this this corresponds to a total of 12 adherence points (i.e. 5+5+2). If the user had adhered completely to the recommended behavior according to the behavior change program 120, then they could have scored a maximum of 5+5+5+2+1=18. Therefore, compared to the maximum adherence score the user has adherence score of approximately 67%.

The adherence score, together with the second sleep data 138 indicating the sleep improvement/impact (e.g. measured using questionnaires, wearable devices and the like), can be used to determine the sleep sensitivity level 146, indicating the personal sleep sensitivity to caffeine of this user. An indication or measure of the improvement of the user's sleep may be obtained using user inputted data or data acquired from the second sensor 106. For example, sleep features or parameters such as the time (in minutes) of sleep onset latency or the amount of time spent asleep may be users as an indication of the sleep improvement. Using the sleep improvement and the adherence level 130, it is possible to determine the sensitivity of a person to caffeine using regression models, for example.

Based on the sensitivity level 146, the adherence level 130 and one or more parameters indicative of the impact of the changed behavior on sleep, the optimizer 132 may adapt the reduction function 122 in an attempt to improve the user's adherence to the recommended behavior. For example, to be able to see an impact on sleep for low sensitivity people (if a reduction on sleep onset latency cannot be seen), the reduction function may advise more cups of coffees to be removed. Examples of parameters indicative of the impact of the changed behavior on sleep include sleep onset latency (SOL), total time asleep, time spent awake during the night, number of waking up events during the night, time spent in rapid eye movement (REM) sleep, time spent in non-REM sleep, time spent in deep sleep, time spent in light sleep and the sleep efficiency, defined for example as the proportion of time spent in bed asleep, or as the difference between the total time awake and the total time asleep during the night or while in bed.

In the example above, the reduction function 122 determines the advised or recommended distribution of coffee (i.e. caffeine) throughout the day based on the user's caffeine intake (dose and scheduling) and on its impact on sleep.

From day to day, the interval training function 124 forces some alternation between a high level (i.e. the user's current or regular consumption ho) and a low level (defined by the reduction function l₀). The baseline coffee intake is taken as the first high level (e.g. 10 cups). The advised behavior derived using reduction function 122 is the low level (e.g. 5 cups). The interval training function 124 will therefore determine a duration and frequency of each interval - e.g. alternating between a day of high intake and a day of low intake for a given period, such as 8 days.

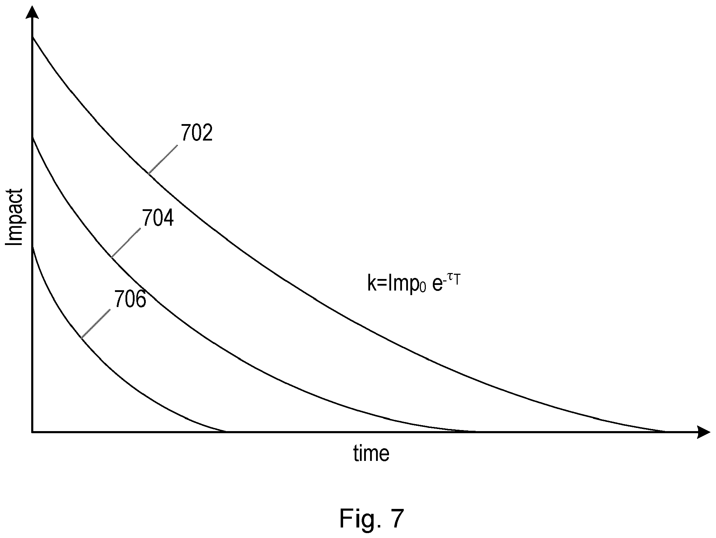

The interval training function 124 determines a pattern of intervals that alternates between these two levels until there is a significant change in the homeostatic state for the user. In one example, this change may be determined using a function that may be referred to as a kappa function. The kappa function may be considered to represent the change in the impact over time of consuming one unit of a substance (e.g. consuming one cup of coffee) following a period of reduced intake. The kappa function describes the decay of the impact of caffeine intake on a given physiological or behavioral parameter (this can be based on sleep parameters, heart rate or blood pressure changes, for example). Kappa changes as the homeostatic states of a person changes as shown, for example, in Fig. 7. Kappa changes with time (for example from one week to another) when the homeostatic state of a person fluctuates. In Fig. 7, line 702 represents the change in impact over time as the user starts to consume their regular high level of coffee intake (e.g. 10 cups) after a period of one week of reduced (i.e. low level) intake (e.g. from 10 cups to 5 cups); line 704 represents the change in impact over time as the user starts to consume their regular high level of coffee intake (e.g. 10 cups) after a period of two weeks of reduced (i.e. low level) intake (e.g. from 10 cups to 5 cups); and line 706 represents the change in impact over time as the user starts to consume their regular high level of coffee intake (e.g. 10 cups) after a period of three weeks of reduced (i.e. low level) intake (e.g. from 10 cups to 5 cups). It is clear that, following a longer period (e.g. three weeks) of reduced caffeine intake, the impact of reverting to a high level of caffeine intake is significantly greater than a relatively shorter period (e.g. one week) of reduced caffeine intake. In some examples, such as the example shown in Fig. 7, Kappa may be represented as an exponential function. However, in other examples, the curve will depend on the user, for example based on the user data 110.
Kappa may be defined as: Kappa=Imp0exp(-tτ)
where Imp is the impact on the user of consuming a coffee, Imp0 is the initial impact of consuming a coffee following a period of reduced consumption, t is the time (x-axis) and τ is the decay rate.

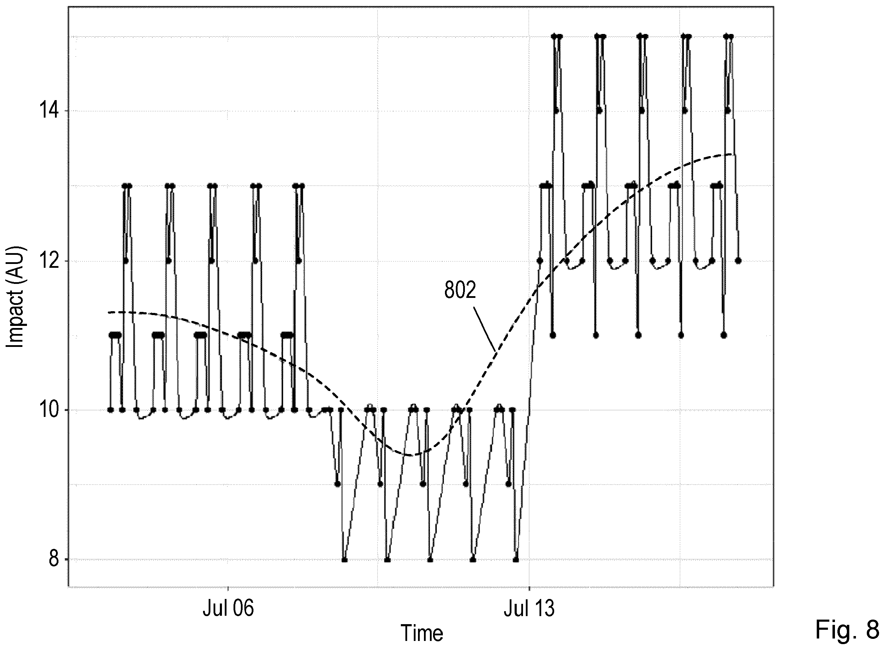

As noted above, alternating between the high intake state and the low intake state (defined by the reduction function 122) may lead to a change in the homeostatic state of a user. The variation of the impact (Imp) from one day to another and from one level to the other can be seen in Fig. 8. Fig. 8 is a graph showing an example of the impact (in arbitrary units, AU) of consuming a coffee as a function of time during each interval. Each data point represents an impact when coffee is consumed. In the example shown, coffee intake is varied between 10 cups per day and 5 cups per day (then back to 10 cups per day). During the first 5-day interval of high intake (10 cups per day), the impact varies throughout the days. Note that, in this example, the impact is displayed per hour, thus, more cups of coffee consumed within the same one-hour period is represented as one data point, such that the data point contains the summation of the impacts of all cups of coffee consumed during that hour. In this example, a moving average (line 802) is indicated. During the second 5-day interval, the user consumes less coffee (5 cups per day) and, during the third 5-day interval, the user consumes coffee at the high level (10 cups per day). The line 802 indicates a moving average impact level from the first to the third interval. In this example, a moving average impact is used; in other examples, other metrics may be used for comparison (e.g. any aggregation of data representative of the change, such as an integral of Imp0(t)). The difference between the value of the moving the average line 802 at the end of the first interval and the third interval represents a delta. The delta function estimates the average change in Imp0 from one level to another (high to high, low to low, high to low, low to high). In this case, the delta function represents the change between the average impact values at the high levels (high to high).

If delta is higher than a given threshold (arbitrarily chosen or based on continuous learning - for example, if the change in the impact is more than a 5 minute increase in the sleep onset latency for 10 cups of coffee intake, then the high level state may be adapted (for example from 10 cups to 8 cups per day). The low level may also be automatically adapted using the corresponding reduction function 122 (for example to 2 cups per day).

If delta does not change after several intervals, the reduction function 122 may be adapted (e.g. to recommend a greater reduction in coffee intake, and advising maintaining the low level of intake for a longer period of time). It is intended that the delta is large enough that the user notices the change, but does not suffer adverse side effects.

The recommended interval training (including adaptation or optimization) determined by the interval training function 124 will be recommended until the target caffeine consumption is achieved or until no further impact is seen.

Embodiments of the present disclosure provide a mechanism by which a change to a user's behavior can be proposed in an effort to improve the user's sleep. The proposed change is provided in the form of a behavior change program which makes use of interval training techniques to change the behavior of the user in a gradual manner aimed at achieving maximum effect with little adverse impact on the user. The initial behavior change program generated using the disclosed embodiments can be optimized following implementation of the program by the user by obtaining data relating to the user after the program has been implemented.

The processor 402, 502 can comprise one or more processors, processing units, multi-core processors or modules that are configured or programmed to control the apparatus 500 in the manner described herein. In particular implementations, the processor 402, 502 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method (200) of determining a change to be made to a behavior affecting sleep of a user, the method comprising:
receiving (202), from at least a first sensor (102) or via a user input, first behavior data (104) relating to a behavior of the user that is to be changed, wherein the behavior affects the user's sleep;
receiving (204), from at least a second sensor (106) or via a user input, first sleep data (108) indicative of a quality of sleep experienced by the user;
receiving (206) user data (110) indicative of at least one physiological parameter relating to the user; and
determining (208), based on the first behavior data (104), the first sleep data (108) and the user data (110), a behavior change program (120) including a change to be made to the behavior of the user and a duration and frequency of each of a plurality of intervals during which the behavior change is to be implemented by the user.

2. A method (200, 300) according to claim 1, further comprising:
providing (304) the behavior change program (120) for display to the user;
receiving (306), from at least the first sensor (102) or via a user input, second behavior data (128) relating to a behavior of the user following the provision of the behavior change program;
determining (308), based on the second behavior data (128), a level of adherence (130) to the behavior change program by the user; and
determining (310), based on the level of adherence, a modification to be made to the behavior change program to increase the level of adherence of the user.

3. A method (200, 300) according to claim 2, further comprising:
receiving (312) daytime functioning data (134) indicative of a quality of daytime functioning by the user;
receiving (314), via a user input (140) or from the second sensor (106), second sleep data (138) indicative of a quality of sleep experienced by the user following the provision of the behavior change program; and
determining (316), based on the daytime functioning data and at least one of the first sleep data and the second sleep data, a user awareness measure indicative of an awareness of the user of an impact of the behavior change program.

4. A method (200, 300) according to claim 3, further comprising:
determining (318), based on the level of adherence (310) and the user awareness measure (142), an indication of the user's susceptibility to behavioral change (144); and
determining (320), based on the second sleep data (138) and the second behavior data (128), a user sensitivity level (146), indicating a sensitivity of the user to the behavior affecting sleep.

5. A method (200, 300) according to claim 4, wherein determining a modification (310) to be made to the behavior change program is further based on the indication of the user's susceptibility to behavioral change and the user sensitivity level.

6. A method (200, 300) according to any of claims 2 to 5, wherein determining a modification (310) to be made to the behavior change program is repeated after a defined number of days.

7. A method (200, 300) according to any of claims 2 to 6, further comprising:
responsive to determining that the level of adherence is below a defined threshold, generating (322) a request to be displayed to the user prompting the user to perform a defined activity.

8. A method (200, 300) according to claim 7, wherein the defined activity is configured to measure daytime functioning data indicative of a quality of daytime functioning by the user.

9. A method (200, 300) according to any of the preceding claims, further comprising:
obtaining (302) data indicative of domain knowledge (116) relating to behavior change techniques;
wherein determining a behavior change program is further based on the domain knowledge data.

10. A computer program product comprising a non-transitory computer-readable medium, the computer-readable medium (404) having computer-readable code embodied therein, the computer-readable code being configured such that, on execution by a suitable computer or processor (402), the computer or processor is caused to perform the method of any of the preceding claims.

11. An apparatus (500) for determining a change to be made to a behavior affecting sleep of a user, the apparatus comprising:
a processor (502) configured to:
receive, from at least a first sensor or via a user input, first behavior data relating to a behavior of the user that is to be changed, wherein the behavior affects the user's sleep;
receive, from at least a second sensor or via a user input, sleep data indicative of a quality of sleep experienced by the user;
receive user data indicative of at least one physiological parameter relating to the user; and
determine, based on the first behavior data, the sleep data and the user data, a behavior change program including a change to be made to the behavior of the user and a duration and frequency of each of a plurality of intervals during which the behavior change is to be implemented by the user.

12. An apparatus (500) according to claim 11, wherein the processor (502) is further configured to:
provide the behavior change program for display to the user;
receive, from at least the first sensor or via a user input, second behavior data relating to a behavior of the user following the provision of the behavior change program;
determine, based on the second behavior data, a level of adherence to the behavior change program by the user; and
determine, based on the level of adherence, a modification to be made to the behavior change program to increase the level of adherence of the user.

13. An apparatus (500) according to claim 12, wherein the processor (502) is further configured to:
receive daytime functioning data indicative of a quality of daytime functioning by the user;
receive, via a user input, second sleep data indicative of a quality of sleep experienced by the user following the provision of the behavior change program; and
determine, based on the daytime functioning data and at least one of the first sleep data and the second sleep data, a user awareness measure indicative of an awareness of the user of an impact of the behavior change program.

14. An apparatus (500) according to claim 13, wherein the processor (502) is further configured to:
determine, based on the level of adherence and the user awareness measure, an indication of the user's susceptibility to behavioral change; and
determine, based on sleep data from the second sensor or via the user input and the daytime functioning data, a user sensitivity level, indicating a sensitivity of the user to the behavior affecting sleep.

15. A system (600) for determining a change to be made to a behavior affecting sleep of a user, the system comprising:
a first sensor (102) configured to measure first behavior data (104) relating to a behavior of the user that is to be changed, wherein the behavior affects the user's sleep;
a second sensor (106) configured to measure sleep data (108) indicative of a quality of sleep experienced by the user;
a display apparatus (126); and
an apparatus (500) according to any of claims 11 to 14.
